(19) Europäisches Patentamt
European Patent Office
Office européen des brevets

(11) EP 3 797 760 A1

(12) **EUROPEAN PATENT APPLICATION**
published in accordance with Art. 153(4) EPC

(43) Date of publication:
**31.03.2021 Bulletin 2021/13**

(21) Application number: **19867399.8**

(22) Date of filing: **30.09.2019**

(51) Int Cl.:
***A61K 8/81*** *(2006.01)* ***A61K 8/06*** *(2006.01)*
***A61K 8/37*** *(2006.01)* ***A61K 8/41*** *(2006.01)*
***A61K 8/49*** *(2006.01)* ***A61Q 17/04*** *(2006.01)*

(86) International application number:
**PCT/JP2019/038447**

(87) International publication number:
**WO 2020/067559 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.09.2018 JP 2018185462**

(71) Applicant: **Kao Corporation**
**Chuo-ku,**
**Tokyo 103-8210 (JP)**

(72) Inventors:
- **OHBA, Chihiro**
  **Wakayama-shi, Wakayama 640-8580 (JP)**
- **HIRONO, Shingo**
  **Tokyo 131-8501 (JP)**
- **ISEKI, Tomokazu**
  **Wakayama-shi, Wakayama 640-8580 (JP)**
- **KAWAMUKAI, Hiroshi**
  **Wakayama-shi, Wakayama 640-8580 (JP)**
- **KODATE, Takashi**
  **Wakayama-shi, Wakayama 640-8580 (JP)**

(74) Representative: **Hoffmann Eitle**
**Patent- und Rechtsanwälte PartmbB**
**Arabellastraße 30**
**81925 München (DE)**

# (54) COSMETICS

(57) A cosmetic material containing a component (A): ionic polymer particles containing structural units derived from (a) one or more hydrophobic monomers selected from the group consisting of styrene and a derivative thereof, a vinyl ester, and a hydrophobic acrylic monomer, and (b) an ionic hydrophilic monomer or a salt thereof, wherein the ratio by mass of (a) to (b), (a)/(b) is 99.5/0.5 to 80/20, and the tetrahydrofuran-insoluble fraction therein is 10% or more and 100% or less, and a component (B): UV protective agent, wherein the ratio by mass of the component (A) to the component (B) is 5/95 to 30/70.

EP 3 797 760 A1

## Description

### Field of the Invention

The present invention relates to a cosmetic material.

### Background of the Invention

UV protective cosmetics are for preventing inflammation or aging of skin to be caused by UV irradiation, and further for preventing skin cancer. Knowledge about UV rays has become widespread in the public, and has changed consumers' consciousness for UV protection, and therefore the recent market for UV protective cosmetics continues to expand.

As skin cosmetics such as UV protective cosmetics, those using an acrylic polymer emulsion for enhancing waterproofness, sebum resistance and adhesiveness to skin are known.

For example, PTL 1 discloses that a soap-free polymer emulsion for cosmetics, which is produced by copolymerizing a specific hydrophobic monomer and a specific hydrophilic monomer and has a glass transition temperature of 5 to -50°C forms a soft film and is excellent in formulation stability (ethanol resistance) and applicability (wrinkling resistance), and discloses that the emulsion is applicable to skin cosmetics such as sunscreens.

PTL 2 discloses a composition containing at least one sunburn protective active agent and at least one dispersion of acrylic polymer particles, in which at least one oil dispersion exists in an amount effective for increasing the waterproofness and/or SPF of the composition.

### Citation List

### Patent Literature

PTL 1: JP 2006-8561 A
PTL 2: JP 2017-538726 A

### Summary of the Invention

The present invention relates to the following [1] to [6].

[1] A cosmetic material containing:
a component (A):

ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the tetrahydrofuran-insoluble fraction therein is 10% or more and 100% or less, and
a component (B): UV protective agent, wherein:

the ratio by mass of the component (A) to the component (B) is 5/95 to 30/70.

[2] A cosmetic material containing:
a component (A1):

ionic polymer particles produced according to a soap-free emulsion polymerization method and containing structural units derived from:

(a) one or more hydrophobic monomers selected from the group consisting of styrene and a derivative thereof, a vinyl ester, and a hydrophobic acrylic monomer, and

(b) an ionic hydrophilic monomer or a salt thereof, wherein:

the ratio by mass of (a) to (b), (a)/(b) is 99.5/0.5 to 80/20, and
a component (B): UV protective agent, wherein:

the ratio by mass of the component (A1) to the component (B) is 5/95 to 30/70.

[3] A UV protection method including a step of applying the cosmetic material of the above [1] or [2] to an object.
[4] A UV protective effect improver containing:
a component (A):
ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:
the ratio by mass of (a) to (b), (a)/(b) is 99.5/0.5 to 80/20, and the tetrahydrofuran-insoluble fraction therein is 10% or more and 100% or less.

[5] A UV protective effect improving method, including blending the UV protective effect improver of the above [4] into a cosmetic material containing a UV protective agent.
[6] Use of a component (A):
ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the tetrahydrofuran-insoluble fraction therein is 10% or more and 100% or less,
as a UV protective effect improver.

Detailed Description of the Invention

[Cosmetic Material (1)]

The cosmetic material of the present invention contains:
a component (A):
ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the tetrahydrofuran-insoluble fraction therein is 10% or more and 100% or less, and
a component (B): UV protective agent, wherein:
the ratio by mass of the component (A) to the component (B) is 5/95 to 30/70.

The cosmetic material of the present invention is based on the finding that use of the component (A), ionic polymer particles satisfying specific requirements and the component (B), UV protective agent combined in a specific ratio dramatically enhances SPF.
SPF is used worldwide as one index to indicate the UV protective effect of a UV protective cosmetic material, and with the recent increase in beauty consciousness and health consciousness, it is desired to further enhance SPF of already-existing UV protective cosmetic materials. In addition, already-existing UV protective cosmetic materials are insufficient in point of uniform applicability and coating film formability and therefore have a problem that the UV protective effect thereof lowers.
An object of the present invention is to provide a cosmetic material capable of preventing reduction in the effect of a UV protective agent and having a further enhanced SPF.

The present inventors have found that a cosmetic material prepared by combining predetermined ionic polymer material and a UV protective agent in a specific ratio can be a cosmetic material having a high SPF.

The cosmetic material of the present invention has a high SPF and is useful as various cosmetic materials such as typically a sunscreen cosmetic material.

The cosmetic material of the present invention has a high UV protective effect and can be applied to, for example, hair and skin. Above all, the cosmetic material of the present invention is preferably a skin cosmetic material. The skin cosmetic material includes a sunscreen cosmetic material, as well as foundation, makeup base, milk, beauty essence, and cream.

The formulation of the cosmetic material is not specifically limited, and any type of formulations is employable, including liquid, foam, paste, cream, and solid.

Preferably, the component (A) in the cosmetic material of the present invention is an oil-in-water type cosmetic material that forms an oil-in-water emulsion.

In general, a cosmetic material containing a UV protective agent can have an increased SPF by increasing the blending amount of the UV protective agent therein. However, from the viewpoint of safety and in consideration of global development, it is desired to enhance SPF by any other method than increasing the blending amount of a UV protective agent.

In addition, it is important that, when applied to an object to be protected from UV such as skin, a cosmetic material containing a UV protective agent can be applied uniformly with no unevenness for enhancing SPF thereof. For example, in the case where an oil-in-water type cosmetic material is applied to the skin and when there occurs a phenomenon that the UV protective agent-containing phase (oily phase) drops in skin depressions, there may be a possibility that the UV protective effect becomes uneven to lower SPF. The cosmetic material of the present invention uses the component (A), ionic polymer particles in which the tetrahydrofuran-insoluble fraction is not less than a predetermined level, and therefore, it is presumed that a coating film formability is good and the viscosity of the UV protective agent-containing phase (oily phase) can be increased to suppress the above-mentioned phenomenon, as another advantageous effect of the cosmetic material of the present invention.

<Component (A): ionic polymer particles>

The cosmetic material of the present invention contains, as the component (A):
ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:
the ratio by mass of (a) to (b), (a)/(b) is 99.5/0.5 to 80/20, and the tetrahydrofuran-insoluble fraction therein is 10% or more and 100% or less.

The cosmetic material of the present invention uses the above-mentioned component (A) as combined with the component (B), UV protective agent, and therefore dramatically enhances SPF thereof.

"Hydrophobic monomer" in this description means a monomer such that the homopolymer thereof has a solubility in water at 20°C of 1% by mass or less, and "hydrophilic monomer" means a monomer such that the homopolymer thereof has a solubility in water at 20°C of more than 1% by mass. Specific examples of the hydrophobic monomer and the hydrophilic monomer are described below.

(Insoluble Fraction)

The tetrahydrofuran (THF)-insoluble fraction (hereinafter also referred to as "THF-insoluble fraction" in the component (A), ionic polymer particles is 10% or more and 100% or less. When the insoluble fraction falls within the range, SPF of the cosmetic material dramatically increases.

From the viewpoint of SPF increase, the THF-insoluble fraction in the component (A) is preferably 20% or more, more preferably 30% or more, even more preferably 40% or more, further more preferably 50% or more, further more preferably 70% or more, further more preferably 80% or more, further more preferably 90% or more, and the upper limit is 100%.

The THF-insoluble fraction in the component (A) can be determined as follows. The ionic polymer particles of the component (A) are dried, the resultant solid is immersed in THF for 2 weeks, and dried, and from the mass ($W_a$) of the solid before immersion in THF and the mass ($W_b$) of the solid after immersion in THF and drying, the THF-insoluble fraction in the component (A) is calculated according to the following expression.

$$\text{THF-insoluble fraction (\%)} = W_b/W_a \times 100$$

Specifically, the THF-insoluble fraction can be determined according to the method described in the section of Examples.

The THF-insoluble fraction in the component (A) can be controlled by selecting the kind of the monomer to constitute the component (A) and selecting the production method for the component (A). More precisely, when the monomer to constitute the component (A) and the ionic polymer particles of the component (A) have tertiary hydrogen that can be abstracted by a radical, hydrogen abstraction and crosslinking may occur and, as a result, the THF-insoluble fraction increases. From this viewpoint, preferably, the monomer to constitute the component (A) contains at least acrylic acid, an acrylate salt or an acrylate ester.

When the ionic polymer particles of the component (A) are produced according to a soap-free emulsion polymerization method to be mentioned below, the THF-insoluble fraction is high and SPF increases. Accordingly, the component (A) is preferably one produced according to a soap-free emulsion polymerization method.

(Hydrophobic Monomer (a))

The component (A) has a structural unit derived from (a) one or more hydrophobic monomers selected from the group consisting of styrene and a derivative thereof, a vinyl ester, and a hydrophobic acrylic monomer (hereinafter may be simply referred to as "hydrophobic monomer (a)").

[Styrene and Derivative thereof]

The styrene and derivatives thereof usable as the hydrophobic monomer (a) include styrene, α-methylstyrene, methylstyrene, butylstyrene, t-butylstyrene, dimethylstyrene, and divinylbenzene. One or more of these can be used. Among these, from the viewpoint of easiness in emulsion polymerization in production of polymer particles, and from availability and economic potential, styrene is preferred.

[Vinyl Ester]

The vinyl ester usable as the hydrophobic monomer (a) includes vinyl esters having an alkyl group or an alkenyl group, such as vinyl acetate, vinyl propionate, vinyl butyrate, vinyl hexanoate, vinyl octanoate, vinyl decanoate, vinyl laurate, vinyl palmitate, and vinyl stearate, and one or more of these can be used. Among these, from the viewpoint of easiness in emulsion polymerization in production of polymer particles, and from availability and economic potential, vinyl acetate is preferred.

[Hydrophobic Acrylic Monomer]

The hydrophobic acrylic monomer usable as the hydrophobic monomer (a) is preferably (meth)acrylate, and is, for example, a (meth)acrylate represented by the following general formula (1), and the homopolymer thereof has a solubility in water at 20°C of 1% by mass or less. In this description, "(meth)acrylic acid" means methacrylic acid or acrylic acid.

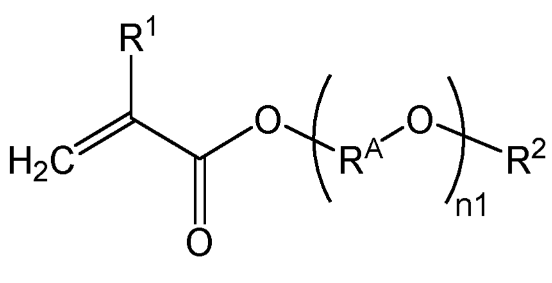
(1)

In the formula (1), $R^1$ represents a hydrogen atom or a methyl group, $R^2$ represents a chainlike aliphatic group having 1 or more and 24 or less carbon atoms, a cycloaliphatic group having 5 or more and 24 or less carbon atoms, an aryl group having 6 or more and 24 or less carbon atoms, or an aralkyl group having 7 or more and 24 or less carbon atoms, which may optionally have a hydroxy group, $R^A$ represents an alkylene group having 2 or more and 4 or less

carbon atoms. n1 represents an integer of 0 or more and 30 or less.

The chainlike aliphatic group in $R^2$ may be any of a straight chainlike aliphatic group or a branched chainlike aliphatic group.

$R^2$ is preferably a chainlike aliphatic group having 1 or more and 24 or less carbon atoms, more preferably an alkyl group having 1 or more and 24 or less carbon atoms, even more preferably an alkyl group having 1 or more and 12 or less carbon atoms, further more preferably an alkyl group having 1 or more and 8 or less carbon atoms, further more preferably an alkyl group having 1 or more and 6 or less carbon atoms.

$R^A$ represents an alkylene group having 2 or more and 4 or less carbon atoms, and is preferably an ethylene group or a propylene group. When n1 is 2 or more, plural $R^A$'s may be the same as or different from each other.

n1 is preferably an integer of 0 or more and 10 or less, more preferably 0.

Among the (meth)acrylate represented by the general formula (1) for the hydrophobic acrylic monomer, an alkyl (meth)acrylate is preferred from the viewpoint of easiness in emulsion polymerization in production of polymer particles, and from availability and economic potential. The carbon number of alkyl is preferably 1 or more and 24 or less, more preferably 1 or more and 12 or less, even more preferably 1 or more and 8 or less, further more preferably 1 or more and 6 or less.

The hydrophobic monomer (a) is, from the viewpoint of controlling the THF-insoluble fraction in the ionic polymer particles of the component (A) to increase SPF, and from the viewpoint of easiness in emulsion polymerization in production of the polymer particles, and from availability and economic potential, preferably one or more selected from the group consisting of styrene, vinyl acetate and a hydrophobic acrylic monomer, more preferably a hydrophobic acrylic monomer, even more preferably an alkyl (meth)acrylate, further more preferably an alkyl (meth)acrylate in which the carbon number of the alkyl is 1 or more and 8 or less, further more preferably 1 or more and 6 or less.

From the viewpoint of controlling the THF-insoluble fraction in the ionic polymer particles of the component (A) to increase SPF, and from the viewpoint of easiness in emulsion polymerization in production of the polymer particles, and from availability and economic potential, the hydrophobic monomer (a) preferably contains 2 or more kinds of hydrophobic monomers. Above all, more preferably, the monomer (a) contains two or more selected from the group consisting of styrene, vinyl acetate, a methacrylate (a1) and an acrylate (a2), even more preferably a methacrylate (a1) and an acrylate (a2).

<<Methacrylate (a1)>>

The methacrylate (a1) includes compounds corresponding to the methacrylates among the above-mentioned hydrophobic acrylic monomers, and are preferably compounds of the general formula (1) where $R^1$ is a methyl group. Above all, from the viewpoint of controlling the THF-insoluble fraction in the ionic polymer particles of the component (A) to increase SPF, from the viewpoint of easiness in emulsion polymerization in production of polymer particles, and from the viewpoint of availability and economic potential, an alkyl methacrylate is preferred, in which the carbon number of the alkyl is preferably 1 or more and 24 or less, more preferably 1 or more and 12 or less, even more preferably 1 or more and 8 or less, further more preferably 1 or more and 6 or less, further more preferably 1 or more and 4 or less.

Specific examples of the methacrylate (a1) include methyl methacrylate, ethyl methacrylate, n-propyl methacrylate, isopropyl methacrylate, n-butyl methacrylate, isobutyl methacrylate, sec-butyl methacrylate, tert-butyl methacrylate, n-hexyl methacrylate, 2-ethylhexyl methacrylate, isooctyl methacrylate, n-decyl methacrylate, isodecyl methacrylate, lauryl methacrylate, cyclohexyl methacrylate, benzyl methacrylate, and isobornyl methacrylate.

Among these, from the viewpoint of controlling the THF-insoluble fraction in the ionic polymer particles of the component (A) to increase SPF, from the viewpoint of easiness in emulsion polymerization in production of polymer particles, and from the viewpoint of availability and economic potential, one or more selected from the group consisting of methyl methacrylate, ethyl methacrylate, n-propyl methacrylate, isopropyl methacrylate, n-butyl methacrylate, isobutyl methacrylate, sec-butyl methacrylate, tert-butyl methacrylate, n-hexyl methacrylate, 2-ethylhexyl methacrylate, isooctyl methacrylate, n-decyl methacrylate, isodecyl methacrylate, and lauryl methacrylate are preferred, one or more selected from the group consisting of methyl methacrylate, ethyl methacrylate, n-propyl methacrylate, isopropyl methacrylate, n-butyl methacrylate, isobutyl methacrylate, sec-butyl methacrylate, tert-butyl methacrylate, n-hexyl methacrylate, 2-ethylhexyl methacrylate, and isooctyl methacrylate are more preferred, one or more selected from the group consisting of methyl methacrylate, ethyl methacrylate, n-propyl methacrylate, isopropyl methacrylate, n-butyl methacrylate, isobutyl methacrylate, sec-butyl methacrylate, tert-butyl methacrylate, and n-hexyl methacrylate are even more preferred, one or more selected from the group consisting of methyl methacrylate, ethyl methacrylate, n-propyl methacrylate, isopropyl methacrylate, and n-butyl methacrylate are further more preferred, and one or more selected from the group consisting of methyl methacrylate, and n-butyl methacrylate are further more preferred.

<<Acrylate (a2)>>

The acrylate (a2) includes compounds corresponding to the acrylates among the above-mentioned hydrophobic acrylic monomers, and are preferably compounds of the general formula (1) where $R^1$ is a hydrogen atom. Above all, from the viewpoint of controlling the THF-insoluble fraction in the ionic polymer particles of the component (A) to increase SPF, from the viewpoint of easiness in emulsion polymerization in production of polymer particles, and from the viewpoint of availability and economic potential, an alkyl acrylate is preferred, in which the carbon number of the alkyl is preferably 1 or more and 24 or less, more preferably 1 or more and 12 or less, even more preferably 1 or more and 8 or less, further more preferably 1 or more and 6 or less, further more preferably 1 or more and 4 or less.
Specific examples of the acrylate (a2) include methyl acrylate, ethyl acrylate, n-propyl acrylate, isopropyl acrylate, n-butyl acrylate, isobutyl acrylate, sec-butyl acrylate, tert-butyl acrylate, n-hexyl acrylate, 2-ethylhexyl acrylate, isooctyl acrylate, n-decyl acrylate, isodecyl acrylate, lauryl acrylate, cyclohexyl acrylate, benzyl acrylate, and isobornyl acrylate.
Among these, from the viewpoint of controlling the THF-insoluble fraction in the ionic polymer particles of the component (A) to increase SPF, from the viewpoint of easiness in emulsion polymerization in production of polymer particles, and from the viewpoint of availability and economic potential, one or more selected from the group consisting of methyl acrylate, ethyl acrylate, n-propyl acrylate, isopropyl acrylate, n-butyl acrylate, isobutyl acrylate, sec-butyl acrylate, tert-butyl acrylate, n-hexyl acrylate, 2-ethylhexyl acrylate, isooctyl acrylate, n-decyl acrylate, isodecyl acrylate, and lauryl acrylate are preferred, one or more selected from the group consisting of methyl acrylate, ethyl acrylate, n-propyl acrylate, isopropyl acrylate, n-butyl acrylate, isobutyl acrylate, sec-butyl acrylate, tert-butyl acrylate, n-hexyl acrylate, and isooctyl acrylate are more preferred, one or more selected from the group consisting of methyl acrylate, ethyl acrylate, n-propyl acrylate, isopropyl acrylate, n-butyl acrylate, isobutyl acrylate, sec-butyl acrylate, tert-butyl acrylate, and n-hexyl acrylate are even more preferred, one or more selected from the group consisting of methyl acrylate, ethyl acrylate, n-propyl acrylate, isopropyl acrylate, and n-butyl acrylate are further more preferred, and n-butyl acrylate is further more preferred.
In the case where the methacrylate (a1) and the acrylate (a2) are used as combined as the hydrophobic monomer (a), the ratio by mass (a1)/(a2) is preferably 5/95 to 99/1, more preferably 10/90 to 90/10, even more preferably 20/80 to 85/15, further more preferably 30/70 to 80/20, further more preferably 30/70 to 70/30, further more preferably 40/60 to 70/30, further more preferably 40/60 to 65/35. Falling within the range facilitates easy control of the THF-insoluble fraction to fall within a desired range and betters various physical properties such as film formability.
The total content of the methacrylate (a1) and the acrylate (a2) in the hydrophobic monomer (a) is, from the viewpoint of controlling the THF-insoluble fraction in the ionic polymer particles of the component (A) to increase SPF, and from the viewpoint of easiness in emulsion polymerization in production of polymer particles, preferably 1% by mass or more, more preferably 5% by mass or more, even more preferably 15% by mass or more, further more preferably 30% by mass or more, further more preferably 50% by mass or more, further more preferably 70% by mass or more, further more preferably 80% by mass or more, further more preferably 90% by mass or more. The upper limit is 100% by mass.

(Ionic Hydrophilic Monomer and Salt thereof (b))

The component (A) has a structural unit derived from ionic hydrophilic monomer or a salt thereof (b). Having the structural unit, the component (A) can form oil-in-water type emulsion particles dispersed in an aqueous medium.
The ionic hydrophilic monomer or a salt thereof includes an anionic group-having anionic hydrophilic monomer or a salt thereof, and a cationic group-having cationic hydrophilic monomer or a salt thereof.
The anionic group in the anionic hydrophilic monomer includes a carboxy group, a sulfonic acid group and a phosphoric acid group, and one or more selected from the group consisting of a carboxy group and a sulfonic acid group are preferred.
Specific examples of the anionic hydrophilic monomer or a salt thereof include a carboxy group-having vinyl compound such as (meth)acrylic acid, maleic acid, fumaric acid, itaconic acid, crotonic acid, and styrene-carboxylic acid, and a salt thereof; a sulfonic acid group-having vinyl compound such as 2-(meth)acrylamide-2-methylpropanesulfonic acid, styrenesulfonic acid, and (meth)acryloyloxyethylsulfonic acid, and a salt thereof; and a phosphoric acid group-having vinyl compound such as vinylphosphonic acid, (meth)acryloyloxyethylphosphoric acid, and a salt thereof. One or more of these can be used.
Specific examples of the cationic hydrophilic monomer and a salt thereof include a dialkylamino group-having (meth)acrylate or (meth)acrylamide such as dimethylaminoethyl (meth)acrylate, diethylaminoethyl (meth)acrylate, dimethylaminopropyl(meth)acrylamide, diethylaminopropyl(meth)acrylamide, and a salt or a quaternary salt thereof; and a diallylamine compound such as diallylmethylamine, and diallylamine, and a salt or a quaternary salt thereof. One or more of these can be used.

Among the above, the ionic hydrophilic monomer or a salt thereof (b) is preferably an anionic hydrophilic monomer or a salt thereof, more preferably a monomer having one or more anionic groups such as a carboxy group and a sulfonic acid group, or a salt thereof, and is, from the viewpoint of controlling the THF-insoluble fraction in the ionic polymer particles of the component (A) to increase SPF, from the viewpoint of easiness in emulsion polymerization in production of polymer particles, and from the viewpoint of availability and economic potential, even more preferably one or more selected from the group consisting of acrylic acid, methacrylic acid, styrenesulfonic acid, and a salt thereof, further more preferably one or more selected from the group consisting of acrylic acid, methacrylic acid, and a salt thereof, further more preferably acrylic acid or a salt thereof.

The component (A) contains structural units derived from a hydrophobic monomer (a), and an ionic hydrophilic monomer or a salt thereof (b), and the ratio by mass of (a) to (b), (a)/(b) is 99.5/0.5 to 80/20. When the ratio by mass of (a)/(b) falls within the range, the THF-insoluble fraction in the ionic polymer particles of the component (A) can be readily controlled to fall within a desired range, and emulsion stability is good.

From the above-mentioned viewpoint, the ratio by mass (a)/(b) is preferably 99/1 to 85/15, more preferably 99/1 to 90/10, even more preferably 98.5/1.5 to 90/10, further more preferably 98/2 to 90/10, further more preferably 95/5 to 90/10.

The component (A) may further have a structural unit derived from any other monomer than the hydrophobic monomer (a) and the ionic hydrophilic monomer or a salt thereof (b), but from the viewpoint of emulsion stability and SPF increase, the total content of the hydrophobic monomer (a) and the ionic hydrophilic monomer or a salt thereof (b) in all the monomers constituting the component (A) is preferably 80% by mass or more, more preferably 90% by mass or more, even more preferably 95% by mass or more, further more preferably 98% by mass or more. The upper limit is 100% by mass.

Further, from the viewpoint of increasing the THF-insoluble fraction by crosslinking to increase SPF, the total content of acrylic acid, acrylate salt and acrylate ester in all the monomers constituting the component (A) is preferably 1% by mass or more, more preferably 5% by mass or more, even more preferably 10% by mass or more, further more preferably 20% by mass or more, further more preferably 30% by mass or more, further more preferably 35% by mass or more, and is, from the same viewpoint and from the viewpoint of film formability, preferably 90% by mass or less, more preferably 80% by mass or less, even more preferably 70% by mass or less, further more preferably 60% by mass or less, further more preferably 50% by mass or less.

(Production Method for Component (A))

The ionic polymer particles of the component (A) can be produced by polymerizing the above-mentioned monomer component and microparticulating the resulting polymer into fine particles. The microparticulating method includes (1) a method of microparticulation with polymerization of a monomer component according to an emulsion polymerization method, a suspension polymerization method or a dispersion polymerization method, and (2) a method of microparticulation including polymerizing a monomer component according to a solution polymerization method to give a polymer, and then microparticulating the resultant polymer according to a phase inversion emulsification method or a suspension method. Among these, from the viewpoint of easiness in production, the method (1) is preferred, and the emulsion polymerization method is more preferred. Among the emulsion polymerization, a soap-free emulsion polymerization method with no surfactant addition is preferred, from the viewpoint of control of the THF-insoluble fraction in the resultant component (A) to increase SPF and from the viewpoint of low skin irritation.

The soap-free emulsion polymerization method is a method of polymerizing a monomer component in emulsion in the presence of a polymerization initiator not using an emulsifier such as a surfactant, a polymer emulsifier, or a reactive surfactant, and can be performed by a known method. In the case of emulsion polymerization, the main component of the solvent to be used is water, and as the case may be, a hydrophilic solvent such as a lower alcohol may be mixed therein. The reaction temperature is set to be not higher than the boiling point of the solvent. The monomer concentration in the reaction system is not specifically limited but is, from the viewpoint of production efficiency and coagulation inhibition, preferably 1 to 60% by mass.

According to the above-mentioned method, the component (A) can be produced in the form of an aqueous dispersion (emulsion) of ionic polymer particles. From the viewpoint of stability and handleability, preferably, the component of this form is blended in the cosmetic material of the present invention.

From the viewpoint of easiness in production and latitude in monomer formulation, the reaction mode is preferably radical polymerization reaction.

The radical polymerization initiator for use in the radical polymerization reaction may be any known compound, and examples thereof include a peroxide initiator such as ammonium persulfate, sodium persulfate, potassium persulfate, benzoyl peroxide, and lauroyl peroxide; and an azo-type initiator such as 2,2'-azobis(2-methylpropionamidine) dihydrochloride, 2,2'-azobis(2,4-dimethylvaleronitrile), and 2,2'-azobisisobutyronitrile. From the viewpoint of soap-free emulsion polymerization, a water-soluble radical polymerization initiator is preferred, and one or more selected from the group

consisting of ammonium persulfate, sodium persulfate and potassium persulfate are more preferred.

The amount of the radical polymerization initiator to be used can be appropriately selected depending on the kind and the concentration of the monomer component, the kind of the radical polymerization initiator, and the polymerization temperature, and is, in general, preferably 0.01% by mass or more and 10% by mass or less relative to the total monomer amount, more preferably 0.1% by mass or more and 5% by mass or less.

The average particle size of the component (A) is, from the viewpoint of SPF increase, preferably 150 nm or more, more preferably 200 nm or more, even more preferably 300 nm or more. Also from the viewpoint of emulsion stability, the average particle size of the component (A) is preferably 800 nm or less, more preferably 700 nm or less, even more preferably 600 nm or less, further more preferably 550 nm or less. Specifically, the range of the average particle size of the component (A) is preferably 150 to 800 nm, more preferably 200 to 700 nm, even more preferably 300 to 600 nm, further more preferably 300 to 550 nm.

In this description, the average particle size of the component (A) means a median diameter (D50). The average particle size is a value measured at 25°C using a laser diffraction/scattering particle size distribution measuring device, and is specifically measured according to the method described in the section of Examples.

When the component (A) is produced according to the soap-free emulsion polymerization method, the THF-insoluble fraction in the component (A) can be increased, SPF can be increased, and further the average particle size can be readily controlled to fall within the above range. In addition, the the soap-free emulsion polymerization method is preferred since use of a surfactant is unnecessary and skin irritation by the component (A) can be thereby reduced.

The content of the component (A) in the cosmetic material is, from the viewpoint of SPF increase, preferably 0.1% by mass or more, more preferably 0.3% by mass or more, even more preferably 0.5% by mass or more, further more preferably 0.8% by mass or more, further more preferably 1.2% by mass or more, and is preferably 5% by mass or less, more preferably 4% by mass or less, even more preferably 3.5% by mass or less, further more preferably 3% by mass or less, further more preferably 2.5% by mass or less. Specifically, the content of the component (A) in the cosmetic material is preferably 0.1 to 5% by mass, more preferably 0.3 to 4% by mass, even more preferably 0.5 to 3.5 % by mass, further more preferably 0.8 to 3% by mass, further more preferably 1.2 to 2.5% by mass.

<Component (B): UV Protective Agent>

The cosmetic material of the present invention contains a component (B), UV protective agent. The UV protective agent includes a UV absorbent and a UV scattering agent. From the viewpoint of achieving an SPF increasing effect as combined with the component (A), a UV absorbent is preferred, and from the viewpoint of achieving the effect of the present invention by the mechanism of action mentioned above, an oil-soluble UV absorbent is more preferred.

The oil-soluble UV absorbent includes a salicylic acid-based UV absorbent, a para-aminobenzoic acid-based UV absorbent, a cinnamic acid-based UV absorbent, a benzophenone-based UV absorbent, a triazine-based UV absorbent, a benzoyl methane-based UV absorbent, and other oil-soluble organic UV absorbents. In this description, "oil-soluble" means that the solubility in water is 1 w/w% or less.

Specific examples of the oil-soluble UV absorbent include:

salicylic acid-based UV absorbents such as homomenthyl salicylate (homosalate, e.g., "Parsol HMS" from DSM Corporation), and octyl salicylate (e.g., "Parsol EHS" from DSM Corporation);
para-aminobenzoic acid-based UV absorbents such as para-aminobenzoic acid, ethyldihydroxypropyl-para-aminobenzoic acid, glyceryl-para-aminobenzoic acid, octyldimethyl-para-aminobenzoic acid, amyl para-dimethylaminobenzoate, and 2-ethylhexyl para-dimethylaminobenzoate;
cinnamic acid-based UV absorbents such as 2-ethylhexyl para-methoxycinnamate (e.g., "Uvinul MC80" from BASF SE), glyceryl dipara-methoxycinnamate mono-2-ethylhexanoate, methyl 2,5-diisopropylcinnamate, methylbis(trimethylsiloxy)silylisopentyl trimethoxycinnamate, and cinnamate mixture of isopropyl/diisopropyl paramethoxycinnamate;
benzophenone-based UV absorbents such as 4-(2-6-glucopyranosiloxy)propoxy-2-hydroxybenzophenone, dihydroxydimethoxybenzophenone, sodium dihydroxydimethoxybenzophenone-disulfonate, 2-hydroxy-4-methoxybenzophenone, 2-hydroxy-4-methoxybenzophenone-5-sulfuric acid, 2,2'-dihydroxy-4-methoxybenzophenone, 2,4-dihydroxybenzophenone, 2,2',4,4'-tetrahydroxybenzophenone, 2,2'-dihydroxy-4,4'-dimethoxybenzophenone, and 2-hydroxy-4-N-octoxybenzophenone;
triazine-based UV absorbents such as 2,4,6-tris[4-(2-ethylhexyloxycarbonyl)anilino]-1,3,5-triazine (hereinafter also referred to as "ethylhexyltriazone", e.g., "Uvinul T150" from BASF SE), and bis(ethylhexyloxyphenol)methoxyphenyltriazine (e.g., "TINOSORB S" from BASF SE);
benzoylmethane-based UV absorbents such as 2-phenyl-benzimidazole-5-sulfuric acid, 4-isopropyldibenzoylmethane, and 4-tert-butyl-4'-methoxydibenzoylmethane (e.g., "Parsol 1789" from DSM Corporation";
octocrylene (e.g., "Parsol 340" from DSM Corporation), 1-(3,4-dimethoxyphenyl)-4,4-dimethyl-1,3-pentanedione,

cinoxate, methyl-o-aminobenzoate, and 3-(4-methylbenzylidene)camphor;

2-ethylhexyl dimethoxybenzylidene-dioxyimidazolidinepropionate (e.g., "Softshade DH" from Ajinomoto Corporation), hexyl diethylaminohydroxybenzoylbenzoate (e.g., "Uvinul Aplus Glanular" from BASF SE), and methylenebisbenzotriazolyltetramethylbutylphenol (e.g., "TINOSORB M" from BASF SE).

One or more of these can be used. From the viewpoint of protection from both UV-A and UV-B, more preferably, 2 or more oil-soluble UV absorbents are used as combined.

From the viewpoint of the effect of UV protection, the component (B) is preferably one or more selected from the group consisting of homomenthyl salicylate (homosalate, e.g., "Parsol HMS" from DSM Corporation), octyl salicylate (e.g., "Parsol EHS" from DSM Corporation), 2-ethylhexyl para-methoxycinnamate (e.g., "Uvinul MC80" from BASF SE), ethylhexyltriazone (e.g., "Uvinul T150" from BASF SE), bis(ethylhexyloxyphenol)methoxyphenyltriazine (e.g., "TINOSORB S" from BASF SE), 4-tert-butyl-4'-methoxydibenzoylmethane (e.g., "Parsol 1789" from DSM Corporation), octocrylene (e.g., "Parsol 340" from DSM Corporation), 2-ethylhexyl dimethoxybenzylidene-dioxyimidazolidinepropionate (e.g., "Softshade DH" from Ajinomoto Corporation), and hexyl diethylaminohydroxybenzoylbenzoate (e.g., "Uvinul Aplus Glanular" from BASF SE), more preferably one or more selected from the group consisting of homomenthyl salicylate, octyl salicylate, 2-ethylhexyl para-methoxycinnamate, ethylhexyltriazone, bis(ethylhexyloxyphenol)methoxyphenyltriazine, 4-tert-butyl-4'-methoxydibenzoylmethane, octocrylene and hexyl diethylaminohydroxybenzoylbenzoate, even more preferably combined use of 2-ethylhexyl paramethoxycinnamate, ethylhexyltriazone, bis(ethylhexyloxyphenol)methoxyphenyltriazine, and hexyl diethylaminohydroxybenzoylbenzoate, or combined use of homomenthyl salicylate, octyl salicylate, 4-tert-butyl-4'-methoxydibenzoylmethane, and octocrylene, and further more preferably combined use of 2-ethylhexyl para-methoxycinnamate, ethylhexyltriazone, bis(ethylhexyloxyphenol)methoxyphenyltriazine, and hexyl diethylaminohydroxybenzoylbenzoate.

The cosmetic material of the present invention contains the component (A) and the component (B), and the ratio by mass of the component (A) to the component (B) is 5/95 to 30/70. Falling within the range, SPF increases dramatically owing to the synergistic effect of the component (A) and the component (B). In addition, the cosmetic material is excellent in usability.

From the above-mentioned viewpoints, the ratio by mass of the component (A) to the component (B) is preferably 10/90 to 25/75, more preferably 10/90 to 20/80.

The content of the component (B) in the cosmetic material is not specifically limited so far as it satisfies the above-mentioned mass ratio, but is, from the viewpoint of SPF increase, preferably 2% by mass or more, more preferably 5% by mass or more, even more preferably 8% by mass or more, further more preferably 10% by mass or more. Also from the viewpoint of usability of the cosmetic material, the content is preferably 40% by mass or less, more preferably 30% by mass or less, even more preferably 20% by mass or less, further more preferably 18% by mass or less, further more preferably 15% by mass or less. Specifically, the range of the content of the component (B) in the cosmetic material is preferably 2 to 40% by mass, more preferably 5 to 30% by mass, even more preferably 8 to 20% by mass, further more preferably 10 to 18% by mass, further more preferably 10 to 15% by mass.

[Aqueous Medium]

The cosmetic material of the present invention can contain an aqueous medium. The aqueous medium includes water; a lower alcohol such as ethanol or isopropyl alcohol; and a low-molecular diol or triol having 6 or less carbon atoms, such as 1,3-butylene glycol, glycerin, ethylene glycol, or propylene glycol. One or more selected from the group consisting of water and a lower alcohol are preferred, and preferably, the aqueous medium contains at least water.

The content of the aqueous medium in the cosmetic material can be appropriately selected depending on the formulation of the cosmetic material and is generally within a range of 1 to 95% by mass. The content of the aqueous medium in the cosmetic material may be a residual part except all the active ingredients in the cosmetic material.

[Other Components]

The cosmetic material of the present invention may optionally contain, in addition to the above-mentioned components, a beauty component and a medically-effective component that are used depending on the use of cosmetic materials, as well as a component generally used in skin cosmetics, within a range not detracting from the object of the present invention. Examples of the components include an antioxidant, a gelling agent, a surfactant, a thickener, an oiling agent, a pH regulator, a stabilizer, a germicide, an anti-inflammatory agent, a preservative, a colorant, a chelating agent, a moisturizer, a pearly agent, ceramides, and a fragrance.

A production method for the cosmetic material of the present invention is not specifically limited. For example, the component (A), the component (B) and other optional components are blended according to the method described in Examples, and mixed using a known stirring device to produce the cosmetic material.

[Cosmetic Material (2)]

The present invention also relates to a cosmetic material containing:
a component (A1):

ionic polymer particles produced according to a soap-free emulsion polymerization method and containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and
a component (B): UV protective agent, wherein:
the ratio by mass of the component (A1) to the component (B) is 5/95 to 30/70. (Hereinafter the cosmetic material may be referred to as "cosmetic material (2)".)

The hydrophobic monomer (a) and the ionic hydrophilic monomer or a salt thereof (b) for use for the component (A1), the ratio by mass of (a) to (b), the component (B), and the preferred embodiments thereof are the same as those described for the cosmetic material (1).
The component (A1) is characterized in that it is produced according to a soap-free emulsion polymerization method, and accordingly, the THF-insoluble fraction in the component (A1) can be increased and, as combined with the component (B), can dramatically increase SPF of the cosmetic material.
The reason why the component (A1) produced according to a soap-free emulsion polymerization method can achieve the above-mentioned effect is not clear, but can be considered to be as follows. In a soap-free emulsion polymerization method, a polymerization initiator attacks the monomer in an aqueous phase to initiate polymerization, and with the progress of the polymerization, the polymer precipitates and coagulates in an aqueous phase to form primary particles. The primary particles act as a production reaction site to further promote the polymerization, and accordingly it is considered that plural radicals existing in the particles may readily crosslink to increase the THF-insoluble fraction in the particles. As opposed to this, in an ordinary emulsion polymerization method, it is presumed that the radical penetration speed into the polymer particles being formed by polymerization and the radical escape speed from them are the same, and only one radical can exist inside the particle and therefore crosslinking can hardly occur in the particles.
The component (A1) can be produced, for example, by polymerizing the hydrophobic monomer (a) and the ionic hydrophilic monomer or a salt thereof (b) for the component (A1) according to the soap-free emulsion polymerization method described relating to the production method for the component (A) hereinabove. From the viewpoint of easiness in production and latitude in monomer formulation, the reaction mode is preferably radical polymerization reaction. From the viewpoint of soap-free emulsion polymerization, a water-soluble radical polymerization initiator is preferably used as the polymerization initiator, and one or more selected from the group consisting of ammonium persulfate, sodium persulfate and potassium persulfate are more preferred.
In general, the amount of the radical polymerization initiator to be used is preferably 0.01% by mass or more and 10% by mass or less, more preferably 0.1% by mass or more and 5% by mass or less relative to the total monomer amount.
Preferred embodiments of the average particle size of the component (A1) and the THF-insoluble fraction in the component are the same as those of the component (A) described in the cosmetic material (1).
The content of the component (A1) in the cosmetic material (2) is, from the viewpoint of SPF increase, preferably 0.1% by mass or more, more preferably 0.3% by mass or more, even more preferably 0.5% by mass or more, further more preferably 0.8% by mass or more, further more preferably 1.2% by mass or more, and is preferably 5% by mass or less, more preferably 4% by mass or less, even more preferably 3.5% by mass or less, further more preferably 3% by mass or less, further more preferably 2.5% by mass or less. Specifically, the range of the content of the component (A1) in the cosmetic material (2) is preferably 0.1 to 5% by mass, more preferably 0.3 to 4% by mass, even more preferably 0.5 to 3.5% by mass, further more preferably 0.8 to 3% by mass, further more preferably 1.2 to 2.5% by mass.
The ratio by mass of the component (A1) to the component (B) in the cosmetic material (2) is 5/95 to 30/70. Falling within the range, SPF dramatically increases owing to the synergistic effect of the component (A1) and the component (B). In addition, the cosmetic material is excellent in usability.
From the above-mentioned viewpoints, the ratio by mass of the component (A1) to the component (B) is preferably 10/90 to 25/75, more preferably 10/90 to 20/80.
The content of the component (B) in the cosmetic material (2) is not specifically limited so far as it satisfies the ratio by mass mentioned above, but is, from the viewpoint of SPF increase, preferably 2% by mass or more, more

preferably 5% by mass or more, even more preferably 8% by mass or more, further more preferably 10% by mass or more. From the viewpoint of usability of the cosmetic material, the content is preferably 40% by mass or less, more preferably 30% by mass or less, even more preferably 20% by mass or less, further more preferably 18% by mass or less, further more preferably 15% by mass or less. Specifically, the range of the content of the component (B) in the cosmetic material (2) is preferably 2 to 40% by mass, more preferably 5 to 30% by mass, even more preferably 8 to 20% by mass, further more preferably 10 to 18% by mass, further more preferably 10 to 15% by mass.

[UV Protection Method]

The present invention also relates to a UV protection method including a step of applying the cosmetic material of the present invention to an object. The method of the present invention is not specifically limited so far as it includes a step of applying the cosmetic material (cosmetic material (1) or cosmetic material (2) of the present invention to an object. The object includes hair and skin, and skin is preferred.

The cosmetic material of the present invention has a high UV protective effect, and therefore can prevent the skin from being inflamed or aged by UV rays, and is further expected to prevent skin cancer.

[UV Protective Effect Improver]

The present invention further provides a UV protective effect improver, containing:
a component (A):
ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:
the ratio by mass of (a) to (b), (a)/(b) is 99.5/0.5 to 80/20, and the tetrahydrofuran-insoluble fraction therein is 10% or more and 100% or less.

The component (A) can be used as a UV protective effect improver, and for example, by blending the UV protective effect improver of the present invention in a cosmetic material containing a UV protective agent such as the component (B), SPF of the resultant cosmetic material can be dramatically increased.

The component (A) and the preferred embodiment thereof are the same as those described for the component (A) in the cosmetic material (1). The component (A) is preferably one produced according to a soap-free emulsion polymerization method, that is, preferably the component (A1) described for the cosmetic material (2).

The content of the component (A) in the UV protective effect improver of the present invention is, from the viewpoint of blending the improver in a cosmetic material containing a UV protective agent for dramatic SPF increase, preferably 1% by mass or more, more preferably 5% by mass or more, even more preferably 10% by mass or more, further more preferably 30% by mass or more, further more preferably 50% by mass or more, further more preferably 70% by mass or more, further more preferably 80% by mass or more, further more preferably 90% by mass or more. The upper limit is 100% by mass.

[UV Protective Effect Improving Method]

The present invention further provides a UV protective effect improving method, including blending a UV protective effect improver that contains the component (A) in a cosmetic material containing a UV protective agent. The UV protective agent includes the same as that of the component (B) described for the cosmetic material (1).

Regarding the blending amount of the UV protective effect improver in the UV protective effect improving method of the present invention, the content of the component (A) in the cosmetic material is, from the viewpoint of SPF increase, preferably 0.1% by mass or more, more preferably 0.3% by mass or more, even more preferably 0.5% by mass or more, further more preferably 0.8% by mass or more, further more preferably 1.2% by mass or more, and is preferably 5% by mass or less, more preferably 4% by mass or less, even more preferably 3.5% by mass or less, further more preferably 3% by mass or less, further more preferably 2.5% by mass or less.

The ratio by mass of the component (A) in the cosmetic material to the UV protective agent is preferably 5/95 to 30/70, more preferably 10/90 to 25/75, even more preferably 10/90 to 20/80. Falling within the range, SPF dramatically increases owing to the synergistic effect of the component (A) and the UV protective agent such as the component (B). In addition, the cosmetic material is excellent in usability.

Regarding the above-mentioned embodiments, the present invention discloses a cosmetic material, a UV protection method, a UV protective effect improver, a UV protective effect improving method, and use of the UV protective

effect improver as mentioned below.

<1> A cosmetic material containing:
a component (A):
ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the tetrahydrofuran-insoluble fraction therein is 10% or more and 100% or less, and
a component (B): UV protective agent, wherein:
the ratio by mass of the component (A) to the component (B) is 5/95 to 30/70.

<2> A cosmetic material containing:
a component (A1):
ionic polymer particles produced according to a soap-free emulsion polymerization method and containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and
a component (B): UV protective agent, wherein:
the ratio by mass of the component (A1) to the component (B) is 5/95 to 30/70.

<3> A UV protective effect improver containing:
a component (A):
ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:
the ratio by mass of (a) to (b), (a)/(b) is 99.5/0.5 to 80/20, and the tetrahydrofuran-insoluble fraction therein is 10% or more and 100% or less.

<4> A UV protective effect improving method, including blending the UV protective effect improver of <3> into a cosmetic material containing a UV protective agent.
<5> Use of a component (A):
ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the tetrahydrofuran-insoluble fraction therein is 10% or more and 100% or less,
as a UV protective effect improver.

<6> The cosmetic material according to <1> or <2>, the UV protective effect improver according to <3>, the UV protective effect improving method according to <4>, or use according to <5>, wherein:

the component (a) is one or more selected from the group consisting of styrene, vinyl acetate and a hydrophobic acrylic monomer, and
the component (b) is an anionic hydrophilic monomer having one or more anionic groups selected from the group consisting of a carboxy group and a sulfonic acid group or a salt thereof.

<7> The cosmetic material, the UV protective effect improver, the UV protective effect improving method or use according to any one of <1> to <6>, wherein the hydrophobic acrylic monomer is a (meth)acrylate represented by the following general formula (1):

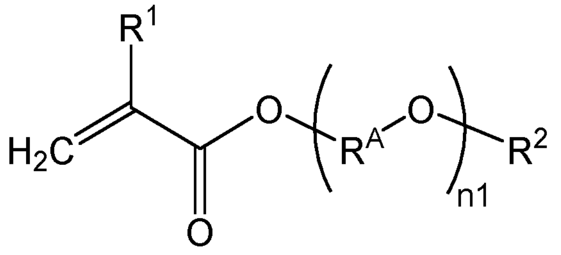

(1)

In the formula (1), $R^1$ represents a hydrogen atom or a methyl group, $R^2$ represents a chainlike aliphatic group having 1 or more and 24 or less carbon atoms, a cycloaliphatic group having 5 or more and 24 or less carbon atoms, an aryl group having 6 or more and 24 or less carbon atoms, or an aralkyl group having 7 or more and 24 or less carbon atoms, which may optionally have a hydroxy group, $R^A$ represents an alkylene group having 2 or more and 4 or less carbon atoms. n1 represents an integer of 0 or more and 30 or less carbon atoms.

<8> The cosmetic material, the UV protective effect improver, the UV protective effect improving method or use according to any one of <1> to <7>, wherein the component (a) contains 2 or more selected from the group consisting of styrene, vinyl acetate, a methacrylate (a1) and an acrylate (a2).

<9> The cosmetic material, the UV protective effect improver, the UV protective effect improving method or use according to <8>, wherein the component (a) contains a methacrylate (a1) and an acrylate (a2).

<10> The cosmetic material, the UV protective effect improver, the UV protective effect improving method or use according to <8> or <9>, wherein the methacrylate (a1) is one or more selected from the group consisting of methyl methacrylate, ethyl methacrylate, n-propyl methacrylate, isopropyl methacrylate, and n-butyl methacrylate.

<11> The cosmetic material, the UV protective effect improver, the UV protective effect improving method or use according to any one of <8> to <10>, wherein the acrylate (a2) is one or more selected from the group consisting of methyl acrylate, ethyl acrylate, n-propyl acrylate, isopropyl acrylate and n-butyl acrylate.

<12> The cosmetic material, the UV protective effect improver, the UV protective effect improving method or use according to any one of <1> to <11>, wherein the component (b) is one or more selected from the group consisting of acrylic acid, methacrylic acid, styrenesulfonic acid, and a salt thereof.

<13> The cosmetic material, the UV protective effect improver, the UV protective effect improving method or use according to <12>, wherein the component (b) is acrylic acid or a salt thereof.

<14> The cosmetic material, the UV protective effect improver, the UV protective effect improving method or use according to any one of <9> to <13>, wherein the ratio by mass of the methacrylate (a1) to the acrylate (a2), (a1)/(a2) is 40/60 to 70/30.

<15> The cosmetic material, the UV protective effect improver, the UV protective effect improving method or use according to any one of <9> to <14>, wherein the total amount of the methacrylate (a1) and the acrylate (a2) in the component (a) is 50 to 100% by mass.

<16> The cosmetic material, the UV protective effect improver, the UV protective effect improving method or use according to any one of <1> to <15>, wherein the ratio by mass (a)/(b) is 98/2 to 90/10.

<17> The cosmetic material, the UV protective effect improver, the UV protective effect improving method or use according to any one of <1> to <16>, wherein the total amount of the hydrophobic monomer (a) and the ionic hydrophilic monomer or a salt thereof (b) in all the monomers constituting the component (A) or the component (A1) is 80 to 100% by mass.

<18> The cosmetic material, the UV protective effect improver, the UV protective effect improving method or use according to any one of <1> to <17>, wherein the total amount of acrylic acid, an acrylate salt and an acrylate ester in all the monomers constituting the component (A) or the component (A1) is 1% by mass or more and 60% by mass or less.

<19> The cosmetic material, the UV protective effect improver, the UV protective effect improving method or use according to any one of <1> to <18>, wherein the average particle size of the component (A) or the component (A1) is 150 to 800 nm.

<20> The cosmetic material according to any one of <1>, <2> and <6> to <19>, wherein the content of the component (A) or the component (A1) in the cosmetic material is 0.1 to 5% by mass.

<21> The cosmetic material according to any one of <1>, <2> and <6> to <20>, wherein the component (B) is one or more selected from the group consisting of homomenthyl salicylate, octyl salicylate, 2-ethylhexyl para-methoxycinnamate, ethylhexyltriazone, bis(ethylhexyloxyphenol)methoxyphenyltriazine, 4-tert-butyl-4'-methoxydibenzoylmethane, octocrylene, 2-ethylhexyl dimethoxybenzylidene-dioxoimidazolidinepropionate, and hexyl diethylaminohydroxybenzoylbenzoate.

<22> The cosmetic material according to any one of <1>, <2> and <6> to <21>, wherein the component (B) is a combination of 2 or more oil-soluble UV absorbents.

<23> The cosmetic material according to any one of <1>, <2> and <6> to <22>, wherein the content of the component (B) in the cosmetic material is 2 to 40% by mass.

<24> The cosmetic material according to any one of <1>, <2> and <6> to <23>, further containing an aqueous medium.

<25> A cosmetic material containing:
a component (A):
ionic polymer particles containing structural units derived from:

(a) 2 or more hydrophobic monomers selected from the group consisting of styrene, vinyl acetate, a methacrylate (a1) and an acrylate (a2), and
(b) acrylic acid or a salt thereof, wherein:

the ratio by mass of (a) to (b), (a)/(b) is 99.5/0.5 to 80/20, and the tetrahydrofuran-insoluble fraction therein is 10% or more and 100% or less, and
a component (B): one or more UV protective agents selected from the group consisting of homomenthyl salicylate, octyl salicylate, 2-ethylhexyl para-methoxycinnamate, ethylhexyltriazone, bis(ethylhexyloxyphenol)methoxyphenyltriazine, 4-tert-butyl-4'-methoxydibenzoylmethane, octocrylene, 2-ethylhexyl dimethoxybenzylidene-dioxoimidazolidinepropionate, and hexyl diethylaminohydroxybenzoylbenzoate, wherein:
the content of the component (A) is 0.1 to 5% by mass,
the content of the component (B) is 2 to 40% by mass, and
the ratio by mass of the component (A) to the component (B) is 5/95 to 30/70.

<26> A cosmetic material containing:
a component (A1):
ionic polymer particles produced according to a soap-free emulsion polymerization method and containing structural units derived from:

(a) 2 or more hydrophobic monomers selected from the group consisting of styrene, vinyl acetate, a methacrylate (a1) and an acrylate (a2), and
(b) acrylic acid or a salt thereof, wherein:

the ratio by mass of (a) to (b), (a)/(b) is 99.5/0.5 to 80/20, and
a component (B): one or more UV protective agents selected from the group consisting of homomenthyl salicylate, octyl salicylate, 2-ethylhexyl para-methoxycinnamate, ethylhexyltriazone, bis(ethylhexyloxyphenol)methoxyphenyltriazine, 4-tert-butyl-4'-methoxydibenzoylmethane, octocrylene, 2-ethylhexyl dimethoxybenzylidene-dioxoimidazolidinepropionate, and hexyl diethylaminohydroxybenzoylbenzoate, wherein:
the content of the component (A) is 0.1 to 5% by mass,
the content of the component (B) is 2 to 40% by mass, and
the ratio by mass of the component (A) to the component (B) is 5/95 to 30/70.

<27> A cosmetic material containing:
a component (A):
ionic polymer particles containing structural units derived from:

(a) 2 or more hydrophobic monomers selected from the group consisting of styrene, vinyl acetate, a methacrylate (a1) and an acrylate (a2), and
(b) acrylic acid or a salt thereof, wherein:

the total amount of acrylic acid, the acrylate salt and the acrylate ester in all the monomer constituting the component (A) is 30% by mass or more and 70% by mass or less,
the ratio by mass of (a) to (b), (a)/(b) is 95/5 to 90/10, and the tetrahydrofuran-insoluble fraction therein is 70% or more and 100% or less, and
a component (B): one or more UV protective agents selected from the group consisting of homomenthyl

salicylate, octyl salicylate, 2-ethylhexyl para-methoxycinnamate, ethylhexyltriazone, bis(ethylhexyloxyphenol)methoxyphenyltriazine, 4-tert-butyl-4'-methoxydibenzoylmethane, octocrylene, 2-ethylhexyl dimethoxybenzylidene-dioxoimidazolidinepropionate, and hexyl diethylaminohydroxybenzoylbenzoate, wherein:

the content of the component (A) is 0.1 to 5% by mass,
the content of the component (B) is 2 to 40% by mass, and
the ratio by mass of the component (A) to the component (B) is 5/95 to 30/70.

<28> A cosmetic material containing:
a component (A):
ionic polymer particles produced according to a soap-free emulsion polymerization method and containing structural units derived from:

(a) 2 or more hydrophobic monomers selected from the group consisting of styrene, vinyl acetate, a methacrylate (a1) and an acrylate (a2), and
(b) acrylic acid or a salt thereof, wherein:

the total content of acrylic acid, the acrylate salt and the acrylate ester in all the monomers constituting the component (A) is 30% by mass or more and 70% by mass or less,
the ratio by mass of (a) to (b), (a)/(b) is 95/5 to 90/10, and
a component (B): one or more UV protective agents selected from the group consisting of homomenthyl salicylate, octyl salicylate, 2-ethylhexyl para-methoxycinnamate, ethylhexyltriazone, bis(ethylhexyloxyphenol)methoxyphenyltriazine, 4-tert-butyl-4'-methoxydibenzoylmethane, octocrylene, 2-ethylhexyl dimethoxybenzylidene-dioxoimidazolidinepropionate, and hexyl diethylaminohydroxybenzoylbenzoate, wherein:

the content of the component (A) is 0.1 to 5% by mass,
the content of the component (B) is 2 to 40% by mass, and
the ratio by mass of the component (A) to the component (B) is 5/95 to 30/70.

<29> A UV protection method including a step of applying the cosmetic material of any one of <1>, <2> and <6> to <28> to an object.

Examples

Hereinunder the present invention is described with reference to Examples, but the present invention is not limited to the scope of Examples. In these Examples, measurement and evaluation were carried out according to the following methods.

(THF-Insoluble Fraction)

The emulsion of polymer particles used in each Example was dried to give a solid, and about 0.5 g of the solid was taken, and immersed in an amount of THF enough to fully immerse the solid at room temperature (25°C) for 2 weeks. After immersion, the liquid was filtered through a 200-mesh screen, and the filtered solid was dried under reduced pressure (-100 kPa) at 80°C for 12 hours. From the mass ($W_a$) of the solid before immersion in THF and the mass ($W_b$) of the solid after immersion in THF and drying, the insoluble fraction was calculated according to the following expression.

$$\text{THF-Insoluble Fraction (\%)} = W_b/W_a \times 100$$

(Average Particle Size of Polymer Particles)

The average particle size (median diameter: D50) of polymer particles was determined, using a laser diffraction/scattering particle size distribution measuring device "LA-920" from HORIBA, Ltd., and using water as a dispersion medium with a relative refractive index: 1.200 to 0.000i.

(in vitro SPF)

On a 5 cm × 5 cm polymethyl methacrylate (PMMA) plate, the cosmetic material of each Example was uniformly applied in an amount of 1.3 mg/$cm^2$, and dried at room temperature (25°C) for 15 minutes.

Using an SPF analyzer ("SPF 290S plus" from Optometricus USA), the transmittance (%) at a wavelength of 350 nm was measured in the center, and at the tips (four points) and the midpoints of four sides (4 points) of a square PMMA plate, totaling 9 points, and the data at 9 points were averaged (N = 3). The SPF value derived from the average value is shown in Table 2 and Table 3. In Table 2, the SPF value of Comparative Example 4 not containing the component (A) is 100, and in Table 3, the SPF value of Comparative Example 5 not containing the component (A) is 100, and based on these, SPF relative values of the others are shown.

Production Example 1 (Production of polymer particles 1)

510 g of ion-exchanged water was put into a 1-liter glass-made separable flask, and stirred in a nitrogen atmosphere for 30 minutes. The flask was heated up to about 70°C, and then after the system therein reached 70°C, a solution prepared by dissolving 1.5 g of ammonium persulfate in 15 g of ion-exchanged water was added thereto. Next, a monomer solution prepared by uniformly mixing 204 g of methyl methacrylate, 87 g of n-butyl acrylate and 9 g of acrylic acid was dropwise added to the system at a constant speed taking 3 hours. After the dropwise addition, this was kept at around 70°C for 1 hour, then heated up to around 75°C and kept as such for 3 hours to attain polymerization and aging. The resultant reaction solution was cooled, and then neutralized with 43.7 g of an aqueous 1 N-sodium hydroxide solution added thereto. Aggregates were removed through screen filtration (200 mesh), and water was added to the filtrate to give a dispersion of polymer particles 1 having an active ingredient concentration of 30% by mass. The THF-insoluble fraction in the polymer particles 1 was 42%, and the average particle size of the particles was 450 nm.

Production Examples 2 to 7, Comparative Production Example 1 (Production of polymer particles 2 to 8)

Dispersions of polymer particles 2 to 8 were produced according to the same method as in Production Example 1 except that the monomers of the quantities and the kinds shown in Table 1 were used. The polymer particles 5 to 7 were prepared to be dispersions thereof having an active ingredient concentration of 20% by mass by adding water thereto. The THF-insoluble fraction in the polymer particles 2 to 8 is shown in Table 1.

Comparative Production Example 2 (Production of polymer particles 9)

510 g of ion-exchange water and 6 g of sodium polyoxyethylene(3) lauryl ether sulfate ("Emal 20C" from Kao Corporation) were put into a 1-liter glass-made separable flask, and stirred in a nitrogen atmosphere for 30 minutes. The flask was heated up to about 70°C, and then after the system therein reached 70°C, a dispersion prepared by dispersing 1.5 g of 4,4'-azobis(4-cyanovaleric acid) ("V-501" from FUJIFILM Wako Pure Chemical Corporation) in 15 g of ion-exchanged water was added thereto. After the system was confirmed to be uniform and transparent, a monomer solution prepared by uniformly mixing 204 g of methyl methacrylate, 87 g of n-butyl acrylate and 9 g of acrylic acid was dropwise added to the reaction vessel at a constant speed taking 3 hours. After the dropwise addition, this was kept at around 70°C for 1 hour, then heated up to around 75°C and kept as such for 3 hours to attain polymerization and aging. The resultant reaction solution was cooled, and then partially neutralized with 43.7 g of an aqueous 1 N-sodium hydroxide solution added thereto. Aggregates were removed through screen filtration (200 mesh), and water was added to the filtrate to give a dispersion of polymer particles 9 having an active ingredient concentration of 30% by mass. The THF-insoluble fraction in the polymer particles 9 is shown in Table 1.

Comparative Production Example 3 (Production of polymer particles 10)

A dispersion of polymer particles 10 was produced according to the same method as in Comparative Production Example 2 except that the monomers of the quantities and the kinds shown in Table 1 were used. The THF-insoluble fraction in the polymer particles 10 is shown in Table 1.

Table 1

| Ingredient (g) | | Production Example | | | | | | | Comparative Production Example | | |
|---|---|---|---|---|---|---|---|---|---|---|---|
| | | 1 | 2 | 3 | 4 | 5 | 6 | 7 | 1 | 2 | 3 |
| No. of Polymer Particles | | 1 | 2 | 3 | 4 | 5 | 6 | 7 | 8 | 9 | 10 |
| (a) Hydrophobic Monomer | (a1-1) methyl methacrylate | 204 | 195 | 153 | 48 | - | - | - | 48 | 204 | 48 |
| | (a1-2) n-butyl methacrylate, | - | - | - | - | 2 | 22 | 29 | 243 | - | - |
| | (a2)n-butyl acrylate | 87 | 87 | 129 | 243 | - | - | - | - | 87 | 243 |
| | styrene | - | - | - | - | 95 | - | 68 | - | - | - |
| | vinyl acetate | - | - | - | - | - | 75 | - | - | - | - |
| (b) Ionic Hydrophilic Monomer | (b1) acrylic acid | 9 | 18 | 18 | 9 | 3 | 3 | 3 | - | 9 | 9 |
| | (b2) methacrylic acid | - | - | - | - | - | - | - | 9 | - | - |
| Polymerization Initiator | ammonium persulfate | 1.5 | 1.5 | 1.5 | 1.5 | 0.5 | 0.5 | 0.5 | 1.5 | - | - |
| | V-501 *1 | - | - | - | - | - | - | - | - | 1.5 | 1.5 |
| Emulsifier | Emal 20C *2 | - | - | - | - | - | - | - | - | 6 | 6 |
| Solvent | ion -exchanged water | 525 | 525 | 525 | 525 | 299 | 299 | 299 | 525 | 525 | 525 |
| Neutralizing Agent | IN NaOH aq | 43.7 | 87.4 | 87.4 | 43.7 | 14.6 | 14.6 | 14.6 | 36.6 | 43.7 | 43.7 |
| (a1)+(a2) in (a) (mass%) | | 100 | 100 | 100 | 100 | 2.1 | 22.7 | 29.9 | 100 | 100 | 100 |
| mass ratio (a1)/(a2) | | 70/30 | 69/31 | 54/46 | 16/84 | 100/0 | 100/0 | 100/0 | 100/0 | 70/30 | 16/84 |
| mass ratio (a)/(b) | | 97/3 | 94/6 | 94/6 | 97/3 | 97/3 | 97/3 | 97/3 | 97/3 | 97/3 | 97/3 |
| Total Amount (mass%) of acrylic acid, acrylate salt and acrylate ester in all monomers constituting polymer | | 32 | 35 | 49 | 84 | 3 | 3 | 3 | 0 | 32 | 84 |
| THF-insoluble Fraction (%) | | 42 | 77 | 100 | 81 | 42 | 20 | 33 | 0 | 5 | 0 |

*1: 4,4'-azobis(4-cyanovaleric acid) ("V-501" from FUJIFILM Wako Pure Chemical Corporation)
*2: sodium polyoxyethylene (3) lauryl ether sulfate ("Emal 20C" from Kao Corporation)

Examples 1 to 7, Comparative Examples 1 to 5 (Production and Evaluation - 1 of oil-in-water type sunscreen cosmetic materials)

With stirring a formulation tank containing pure water in an amount shown in Table 2 with a disperser, a preservative, a viscosity regulator, a stabilizer and a 95° synthetic alcohol shown in Table 2 were added thereto at room temperature, and further, a pH regulator was added thereto to prepare an aqueous phase. On the other hand, a component (B), an oily agent of alkyl (C12-C15) benzoate and a gelling agent of dextrin palmitate were stirred with heating at about 85°C to prepare an oily phase. The oily phase was added to the aqueous phase, stirred, and a dispersion of polymer particles of a component (A) or any other than the component (A) (in Table 2, expressed as "component (A')") was added and stirred to give an oil-in-water type sunscreen cosmetic material having a composition shown in Table 2. The component (A) and the component (A') was blended in an amount of 2% by mass as a concentration of the active ingredient in the cosmetic material.

The resultant cosmetic materials were tested for in vitro SPF evaluation according to the above-mentioned method. The results are shown in Table 2.

Table 2

| Ingredient (mass%) | | Example | | | | | | | Comparative Example | | | | |
|---|---|---|---|---|---|---|---|---|---|---|---|---|---|
| | | 1 | 2 | 3 | 4 | 5 | 6 | 7 | 1 | 2 | 3 | 4 | 5 |
| Dispersion of Component (A) | dispersion of polymer particles 1 (THF-insoluble fraction, 42%) | 6.7 | | | | | | | | | | | |
| | dispersion of polymer particles 2 (THF-insoluble fraction, 77%) | | 6.7 | | | | | | | | | | |
| | dispersion of polymer particles 3 (THF-insoluble fraction, 100%) | | | 6.7 | | | | | | | | | |
| | dispersion of polymer particles 4 (THF-insoluble fraction, 81%) | | | | 6.7 | | | | | | | | |
| | dispersion of polymer particles 5 (THF-insoluble fraction, 42%) | | | | | 10.0 | | | | | | | |
| | dispersion of polymer particles 6 (THF-insoluble fraction, 20%) | | | | | | 10.0 | | | | | | |
| | dispersion of polymer particles 7 (THF-insoluble fraction, 33%) | | | | | | | 10.0 | | | | | |
| Dispersion of Component (A') | dispersion of polymer particles 8 (THF-insoluble fraction, 0%) | | | | | | | | 6.7 | | | | |
| | dispersion of polymer particles 9 (THF-insoluble fraction, 5%) | | | | | | | | | 6.7 | | | |
| | dispersion of polymer particles 10 (THF-insoluble fraction, 0%) | | | | | | | | | | 6.7 | | |
| | Yodosol GH810F *1 (THF-insoluble fraction, 0%) | | | | | | | | | | | 4.4 | |

(continued)

| Ingredient (mass%) | | Example | | | | | | | Comparative Example | | | | |
|---|---|---|---|---|---|---|---|---|---|---|---|---|---|
| | | 1 | 2 | 3 | 4 | 5 | 6 | 7 | 1 | 2 | 3 | 4 | 5 |
| Component (B) | Uvinul MC80 *2 | 8.5 | 8.5 | 8.5 | 8.5 | 8.5 | 8.5 | 8.5 | 8.5 | 8.5 | 8.5 | 8.5 | 8.5 |
| | Uvinul Aplus Glanular *3 | 1 | 1 | 1 | 1 | 1 | 1 | 1 | 1 | 1 | 1 | 1 | 1 |
| | TINOSORB S *4 | 2 | 2 | 2 | 2 | 2 | 2 | 2 | 2 | 2 | 2 | 2 | 2 |
| | Uvinul T-150 *5 | 0.5 | 0.5 | 0.5 | 0.5 | 0.5 | 0.5 | 0.5 | 0.5 | 0.5 | 0.5 | 0.5 | 0.5 |
| Oily Agent | Finsolv TN *6 | 3 | 3 | 3 | 3 | 3 | 3 | 3 | 3 | 3 | 3 | 3 | 3 |
| Gelling Agent | Rheopearl KL2 *7 | 0.75 | 0.75 | 0.75 | 0.75 | 0.75 | 0.75 | 0.75 | 0.75 | 0.75 | 0.75 | 0.75 | 0.75 |
| Preservative | Hi-Solv EPH *8 | 0.4 | 0.4 | 0.4 | 0.4 | 0.4 | 0.4 | 0.4 | 0.4 | 0.4 | 0.4 | 0.4 | 0.4 |
| Viscosity Regulator | PEMULEN TR-1 *9 | 0.4 | 0.4 | 0.4 | 0.4 | 0.4 | 0.4 | 0.4 | 0.4 | 0.4 | 0.4 | 0.4 | 0.4 |
| Stabilizer | Kurewat N *10 | 0.01 | 0.01 | 0.01 | 0.01 | 0.01 | 0.01 | 0.01 | 0.01 | 0.01 | 0.01 | 0.01 | 0.01 |
| pH Regulator | liquid KOH (48%) | 0.2 | 0.2 | 0.2 | 0.2 | 0.2 | 0.2 | 0.2 | 0.2 | 0.2 | 0.2 | 0.2 | 0.2 |
| Aqueous Medium | 95° synthetic alcohol *11 | 10 | 10 | 10 | 10 | 10 | 10 | 10 | 10 | 10 | 10 | 10 | 10 |
| | pure water | 66.54 | 66.54 | 66.54 | 66.54 | 63.24 | 63.24 | 63.24 | 66.54 | 66.54 | 66.54 | 68.84 | 73.24 |
| Total | | 100 | 100 | 100 | 100 | 100 | 100 | 100 | 100 | 100 | 100 | 100 | 100 |
| Mass Ratio (A)/(B) (*in Comparative Examples 1 to 4, (A')/(B)) | | 14/86 | 14/86 | 14/86 | 14/86 | 14/86 | 14/86 | 14/86 | 14/86 | 14/86 | 14/86 | 14/86 | 0/100 |
| Evaluation Results | in vitro SPF | 53.6 | 60.0 | 85.2 | 46.0 | 56.5 | 54.8 | 47.0 | 32.6 | 37.8 | 22.3 | 24.9 | 32.1 |
| | SPF relative value | 167 | 187 | 265 | 143 | 176 | 171 | 146 | 101 | 118 | 69 | 77 | 100 |

The formulation ingredients in Table 2 are as follows.

*1 Alkyl acrylate copolymer: Yodosol GH810F (from Akzo Nobel N.V., active ingredient amount: 46% by mass)
*2 2-Ethylhexyl para-methoxycinnamate: Uvinul MC80 (from BASF SE)
*3 Hexyl diethylaminohydroxybenzoylbenzoate: Uvinul Aplus Glanular (from BASF SE)
*4 Bis(ethylhexyloxyphenol)methoxyphenyltriazine: TINOSORB S (from BASF SE)
*5 Ethylhexyltriazone: Uvinul T-150 (from BASF SE)
*6 Alkyl (C12-15) benzoate: Finsolv TN (from Innospec Active Chemicals LLC)
*7 Dextrin palmitate: Rheopearl KL2 (from Chiba Flour Milling Co., Ltd.)
*8 Phenoxyethanol: Hisolv EPH (from TOHO Chemical Industry Co., Ltd.)
*9 (Acrylates/alkyl arylate (C10-30)) cross polymer: PEMULEN TR-1 (from Lubrizol Advanced Materials, Inc.)
*10 EDTA·2Na·$2H_2O$: Kurewat N (from Nagase ChemteX Corporation)
*11 Ethanol: 95° synthetic alcohol (from Japan Synthetic Alcohol Co., Ltd.)

From Table 2, it is known that the cosmetic materials of Examples 1 to 7 have increased SPF than the cosmetic materials of Comparative Examples 1 to 5 in which the ionic polymer particles used have a low THF-insoluble fraction. Comparative Example 2 uses the polymer particles 9, which have the same monomer formulation as that of the polymer particles 1, but were produced not according to a soap-free emulsion polymerization method but through emulsion polymerization using an emulsifier and an azo-type radical polymerization initiator. Comparative Example 3 uses the polymer particles 10, which have the same monomer formulation as that of the polymer particles 4, but were produced not according to a soap-free emulsion polymerization method but through emulsion polymerization using an emulsifier and an azo-type radical polymerization initiator. However, the polymer particles 9 and 10 have a low THF-insoluble fraction, and in comparison between Example 1 and Comparative Example 2 and between Example 4 and Comparative Example 3, the cosmetic materials using these polymer particles are all poor in the effect of increasing SPF.

Example 8, Comparative Example 6 (Production and Evaluation - 2 of oil-in-water type sunscreen cosmetic materials)

While stirring a formulation tank containing pure water in an amount shown in Table 3 with a disperser, a preservative, a viscosity regulator, a stabilizer and a 95° synthetic alcohol shown in Table 3 were added thereto at room temperature, and further, a pH regulator was added thereto to prepare an aqueous phase. On the other hand, a component (B), an oily agent of alkyl (C12-C15) benzoate and a gelling agent of dextrin palmitate were stirred with heating at about 85°C to prepare an oily phase. The oily phase was added to the aqueous phase, stirred, and then a dispersion of a component (A) was added or not added, this was further stirred to give an oil-in-water type sunscreen cosmetic material having a composition shown in Table 3. The component (A) was blended in an amount of 2% by mass as a concentration of the active ingredient in the cosmetic material.

The resultant cosmetic materials were tested for in vitro SPF evaluation according to the above-mentioned method. The results are shown in Table 3.

Table 3

| Ingredient (mass%) | | Example | Comparative Example |
|---|---|---|---|
| | | 8 | 6 |
| Dispersion of Component (A) | dispersion of polymer particles 1 (THF-insoluble fraction, 42%) | 6.7 | - |
| Component (B) | Parsol 1789 *12 | 2 | 2 |
| | Parsol EHS *13 | 5 | 5 |
| | Parsol HMS *14 | 5 | 5 |
| | Parsol 340 *15 | 5 | 5 |
| Oily Agent | Finsolv TN *6 | 3 | 3 |
| Gelling Agent | Rheopearl KL2 *7 | 0.75 | 0.75 |
| Preservative | Hisolv EPH *8 | 0.4 | 0.4 |
| Viscosity Regulator | PEMULEN TR-1 *9 | 0.4 | 0.4 |

(continued)

| Ingredient (mass%) | | Example | Comparative Example |
|---|---|---|---|
| | | 8 | 6 |
| Stabilizer | Kurewat N *10 | 0.01 | 0.01 |
| pH Regulator | liquid KOH (48%) | 0.2 | 0.2 |
| Aqueous Medium | 95° synthetic alcohol *11 | 10 | 10 |
| | pure water | 61.54 | 68.24 |
| Total | | 100 | 100 |
| Ratio by Mass (A)/(B) | | 11/89 | 0/100 |
| Evaluation Results | in vitro SPF | 13.5 | 6.8 |
| | SPF Relative Value | 199 | 100 |

Among the formulation ingredients in Table 3, the ingredients except those described in Table 2 are as follows.

*12 4-Tert-butyl-4'-methoxydibenzoylmethane: Parsol 1789 (from DSM Corporation)
*13 Octyl salicylate: Parsol EHS (from DSM Corporation)
*14 Homosalate: Parsol HMS (from DSM Corporation)
*15 Octocrylene: Parsol 340 (from DSM Corporation)

From Table 3, it is known that the cosmetic material of Example 8 has a higher SPF than the cosmetic material of Comparative Example 6 not using a component (A).

Industrial Applicability

The cosmetic material of the present invention has a high SPF and is useful as various cosmetic materials typically including sunscreen cosmetic materials.

## Claims

1. A cosmetic material comprising:
a component (A):
ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the tetrahydrofuran-insoluble fraction therein is 10% or more and 100% or less, and
a component (B): UV protective agent, wherein:
the ratio by mass of the component (A) to the component (B) is 5/95 to 30/70.

2. The cosmetic material according to claim 1, wherein the hydrophobic monomer (a) is one or more selected from the group consisting of styrene, vinyl acetate and a hydrophobic acrylic monomer.

3. The cosmetic material according to claim 2, wherein the hydrophobic monomer (a) is a hydrophobic acrylic monomer.

4. The cosmetic material according to any one of claims 1 to 3, wherein the hydrophobic acrylic monomer is a (meth)acrylate represented by the following general formula (1):

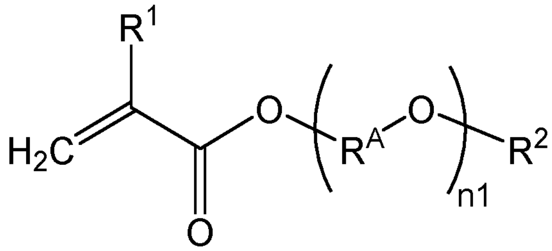

( 1 )

wherein $R^1$ represents a hydrogen atom or a methyl group, $R^2$ represents a chainlike aliphatic group having 1 or more and 24 or less carbon atoms, a cycloaliphatic group having 5 or more and 24 or less carbon atoms, an aryl group having 6 or more and 24 or less carbon atoms, or an aralkyl group having 7 or more and 24 or less carbon atoms, which may optionally have a hydroxy group, $R^A$ represents an alkylene group having 2 or more and 4 or less carbon atoms, and n1 represents an integer of 0 or more and 30 or less.

**5.** The cosmetic material according to any one of claims 1 to 4, wherein the hydrophobic monomer (a) comprises a methacrylate (a1) and an acrylate (a2).

**6.** The cosmetic material according to any one of claims 1 to 5, wherein the ionic hydrophilic monomer or a salt thereof (b) is an anionic hydrophilic monomer having one or more anionic groups selected from the group consisting of carboxy group and a sulfonic acid group, or a salt thereof.

**7.** The cosmetic material according to claim 6, wherein the anionic hydrophilic monomer or a salt thereof is one or more selected from the group consisting of acrylic acid, methacrylic acid, styrenesulfonic acid, and a salt thereof.

**8.** The cosmetic material according to any one of claims 1 to 7, wherein the component (A) is an oil-in-water type cosmetic material that forms an oil-in-water type emulsion.

**9.** A cosmetic material comprising:
a component (A1):
ionic polymer particles produced according to a soap-free emulsion polymerization method and containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and
a component (B): UV protective agent, wherein:
the ratio by mass of the component (A1) to the component (B) is 5/95 to 30/70.

**10.** A UV protection method comprising a step of applying the cosmetic material of any one of claims 1 to 9 to an object.

**11.** A UV protective effect improver comprising:
a component (A):
ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:
the ratio by mass of (a) to (b), (a)/(b) is 99.5/0.5 to 80/20, and the tetrahydrofuran-insoluble fraction therein is 10% or more and 100% or less.

**12.** A UV protective effect improving method, comprising blending the UV protective effect improver of claim 11 into a cosmetic material containing a UV protective agent.

**13.** Use of a component (A):

ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the tetrahydrofuran-insoluble fraction therein is 10% or more and 100% or less,
as a UV protective effect improver.

**INTERNATIONAL SEARCH REPORT**

International application No. PCT/JP2019/038447

A. CLASSIFICATION OF SUBJECT MATTER
Int.Cl. A61K8/81(2006.01)i, A61K8/06(2006.01)i, A61K8/37(2006.01)i, A61K8/41(2006.01)i, A61K8/49(2006.01)i, A61Q17/04(2006.01)i

According to International Patent Classification (IPC) or to both national classification and IPC

B. FIELDS SEARCHED

Minimum documentation searched (classification system followed by classification symbols)
Int.Cl. A61K8/81, A61K8/06, A61K8/37, A61K8/41, A61K8/49, A61Q17/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 05-017320 A (KAO CORP.) 26 January 1993, paragraphs [0007]-[0011], [0019]-[0020], [0023]-[0024], [0034] (Family: none) | 1-13 |
| A | JP 2001-064118 A (KOSÉ CORPORATION) 13 March 2001, paragraphs [0005], [0008], [0011] (Family: none) | 1-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 December 2019 (16.12.2019) | 07 January 2019 (07.01.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/038447

C (Continuation). DOCUMENTS CONSIDERED TO BE RELEVANT

| Category* | Citation of document, with indication, where appropriate, of the relevant passages | Relevant to claim No. |
|---|---|---|
| A | JP 04-103509 A (KAO CORP.) 06 April 1992, page 2, upper left column, line 10 to lower right column, line 13, page 3, lower left column, line 17 to lower right column, line 11, synthesis example 1 (Family: none) | 1-13 |
| A | JP 2012-525334 A (MSD CONSUMER CARE, INC.) 22 October 2012, claims 1, 19, paragraphs [0039], [0053], [0056] & US 2010/0272657 A1, paragraphs [0037], [0051], [0054], claims 1, 19 & WO 2010/129215 A2 & EP 2424622 A2 | 1-13 |
| A | JP 2005-170940 A (NATIONAL STARCH AND CHEMICAL INVESTMENT HOLDING CORPORATION) 30 June 2005, claims, paragraphs [0010], [0015]-[0016], [0019]-[0020] & US 2005/0112080 A1, paragraphs [0010], [0015]-[0016], [0019]-[0022], claims 1-26 & EP 1535938 A1 & CN 1660030 A | 1-13 |
| A | JP 2000-351721 A (F. HOFFMANN-LA ROCHE AG) 19 December 2000, claims 1, 5-6, 8-9, paragraphs [0006], [0016] & US 6338838 B1 & EP 1051963 A1, paragraphs [0006], [0016], claims 1, 5-6, 8-9 & CN 1274577 A & KR 10-2000-0077250 A | 1-13 |
| A | JP 2016-521679 A (L'OREAL) 25 July 2016, claims 1, 9, 11, 13, paragraphs [0136]-[0165], [0202] & WO 2014/203913 A1, page 17, line 11 to page 20, line 25, page 24, line 49 to page 25, line 5, claims 1, 9, 11, 13 | 1-13 |
| A | WO 2017/058404 A1 (ROHM AND HAAS COMPANY) 06 April 2017, page 10, line 9 to page 11, line 11, page 16, line 26 to page 17, line 2, claims 1, 5-6 & JP 2018-531222 A & US 2018/0243186 A1 & CN 107949367 A | 1-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006008561 A
- JP 2017538726 A